# FASCICULE DE BREVET EUROPEEN

(11) **EP 0 968 222 B1**
(45) Date de publication et mention de la délivrance du brevet: **12.06.2002**
(21) Numéro de dépôt: 98912554.7
(22) Date de dépôt: 26.02.1998
(51) Int. Cl.: C07H 17/08, A61K 31/70

(54) **NOUVEAUX DERIVES DE L'ERYTHROMYCINE, LEUR PROCEDE DE PREPARATION ET LEUR APPLICATION COMME MEDICAMENTS**
ERYTHROMYCIN-DERIVATE, VERFAHREN ZU IHRER HERSTELLUNG UND IHRE VERWENDUNG ALS ARZNEIMITTEL
NOVEL ERYTHROMYCIN DERIVATIVES, METHOD OF PREPARATION AND APPLICATION AS MEDICINES

(30) Priorité: 27.02.1997 FR 9702352
(43) Date de publication de la demande: 05.01.2000
(73) Titulaire: Aventis Pharma S.A., 92160 Antony (FR)
(72) Inventeur: AGOURIDAS, Constantin, F-94130 Nogent sur Marne (FR); CHANTOT, Jean-François, F-94130 Nogent sur Marne (FR); DENIS, Alexis, F-75011 Paris (FR); FROMENTIN, Claude, F-75019 Paris (FR); PEJAC, Jean-Marie, F-93140 Bondy (FR)
(86) Numéro de dépôt international: FR9800378
(87) Numéro de publication internationale: WO9838199

(56) Documents cités:
- EP-A- 0 487 411
- US-A- 4 349 545
- US-A- 5 439 889

## Description

L'invention concerne de nouveaux dérivés de l'érythromycine, leur procédé de préparation et leur application comme médicaments.

Le document EP-A-0487411 décrit notamment des dérivés de l'érythromycine comportant en position 9 une fonction oxime substituée et formant en position 11, 12 une fonction carbamate cyclique dont l'atome d'azote peut être substitué par un radical alkylène amino lui-même éventuellement substitué. Les dérivés en question ne sont donc pas des dérivés dont l'atome d'azote de la fonction carbamate forme un groupe hydrazono avec son substituant.

L'invention a pour objet les composés de formule (I) : 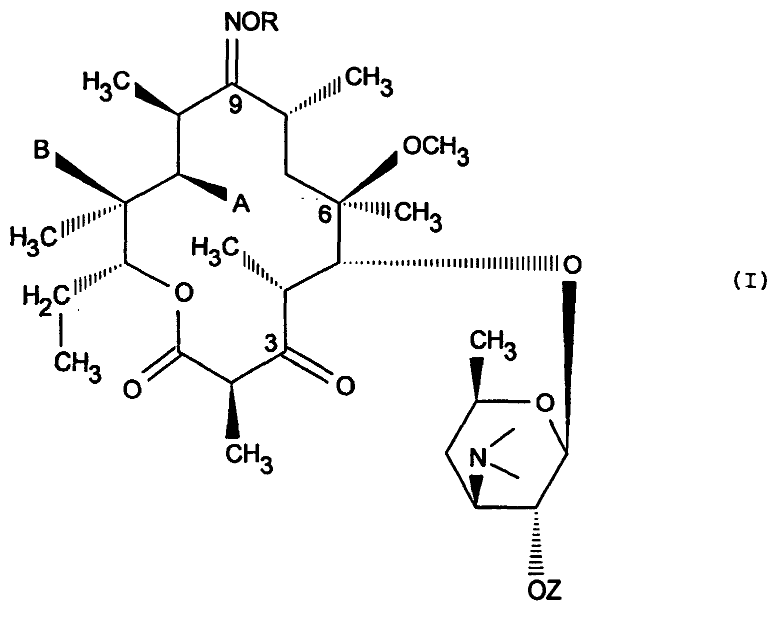 dans lesquels A et B forment avec les atomes auxquels ils sont liés un groupement : 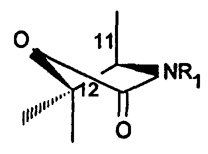
- R₁ représente un radical NH₂ ou un radical NH-alkyle, NH-alkényle ou NH-alkynyle renfermant jusqu'à 18 atomes de carbone ou un radical NH(CH₂)ₙAr ou N=CH(CH₂)ₙAr dans lequel n représente un nombre entier compris entre 1 et 6, et Ar représente un radical aryle ou hétéroaryle éventuellement substitué, Z représente un atome d'hydrogène ou le reste d'un acide carboxylique renfermant jusqu'à 18 atomes de carbone, et R représente :
   - un atome d'hydrogène,
   - un radical hétérocyclique renfermant au moins un atome d'azote et éventuellement un autre hétéroatome, mono ou bicyclique, saturé ou insaturé, aromatique ou non aromatique comportant jusqu'à 12 chaînons, éventuellement substitué sur l'atome d'azote,
   - un radical alkyle, alkényle ou alkynyle linéaire, ramifié ou cyclique, renfermant jusqu'à 18 atomes de carbone éventuellement substitué par un ou plusieurs groupements :
      . hydroxyle,
      . halogène,
      . cyano,
      . nitro,
      . amidinyle,
      . guanidinyle,
      . hétérocyclique, tel que défini précédemment,
      . alkyloxy, alkényloxy ou alkynyloxy ayant au plus 6 atomes de carbone,
      . alkylthio, alkénylthio ou alkynylthio ayant au plus 6 atomes de carbone, l'atome de soufre étant éventuellement oxydé en sulfoxyde ou en sulfone,
      . aryle, aralkyle,
      . aryloxy, aralkyloxy,
      . arylthio, aralkylthio, l'atome de soufre étant éventuellement oxydé en sulfoxyde ou en sulfone,
      . 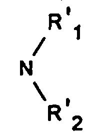 dans lequel :
         **ou bien R'**_{**1**} **et R'**_{**2**} identiques ou différents représentent un atome d'hydrogène, un radical alkyle, alkényle ou alkynyle, linéaire, ramifié ou cyclique renfermant jusqu'à 18 atomes de carbone, un radical aryle ou aralkyle, chacun de ces radicaux R'₁ et R'₂ étant éventuellement substitué par un ou plusieurs radicaux hydroxy, alkyloxy, alkényloxy, alkynyloxy, alkylthio, alkénylthio ou alkynylthio renfermant jusqu'à 8 atomes de carbone, amino, monoalkylamino renfermant jusqu'à 4 atomes de carbone, dialkylamino renfermant jusqu'à 8 atomes de carbone, cyano, carboxyle libre, estérifié ou salifié, acyle ou carbamoyle, renfermant jusqu'à 8 atomes de carbone, par un radical Si(alc)₃ ou Si(Oalc)₃ dans lequel alc représente un radical alkyle renfermant jusqu'à 4 atomes de carbone, par un radical hétérocyclique tel que défini précédemment,
         **ou bien R'**_{**1**} **et R'**_{**2**} forment ensemble avec l'atome d'azote auquel ils sont liés un radical hétérocycle mono ou bicyclique, renfermant éventuellement un autre hétéroatome, saturé ou insaturé, aromatique ou non aromatique, comportant jusqu'à 12 chaînons ;
      . un groupement ammonium quaternaire,
      . 1,2-époxyéthyle ou 2,2-diméthyl 1,2-époxyéthyle ou un radical résultant de l'ouverture de ce groupement par un réactif nucléophile,
      . 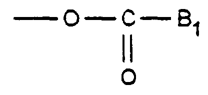 dans lequel B₁ représente soit un radical alkyle ou alkyloxy ayant au plus 6 atomes de carbone, soit un radical aryle, aralkyle, aryloxy ou aralkyloxy,
      . formyle libre ou protégé, carboxyle libre, estérifié ou salifié, thiocyanate, acyle ou carbamoyle,
      . (CH₂)ₙR', R' représentant le reste d'un acide aminé, et n représentant un nombre entier compris entre 0 et 6,
ainsi que les sels d'addition avec les acides des composés de formule (I).

Comme exemple de sels d'addition des présents dérivés avec les acides minéraux ou organiques, on peut citer les sels formés avec les acides acétique, propionique, trifluoroacétique, maléique, tartrique, méthanesulfonique, benzènesulfonique, p-toluènesulfonique, chlorhydrique, bromhydrique, iodhydrique, sulfurique, phosphorique et spécialement les acides stéarique, éthylsuccinique ou laurylsulfurique.

Dans la définition des produits de l'invention :
- le radical hétérocyclique est de préférence le radical pyrrolyle, pyrrolidinyle, pyridyle, pyrazinyle, pyrimidyle, pipéridinyle, pipérazinyle, quinuclidinyle, oxazolyle, isoxazolyle, morpholinyle, indolyle, imidazolyle, benzimidazolyle, triazolyle, thiazolyle, azétidinyle, aziridinyle. On peut naturellement citer de préférence les radicaux hétérocycliques mentionnés ci-après dans la partie expérimentale,
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, cyclobutyle, cyclopentyle ou cyclohexyle,
- l'halogène est de préférence le fluor ou le chlore, ou le brome,
- le radical aryle est de préférence le radical phényle,
- le radical aralkyle est de préférence un radical (C₆H₅)-(CH₂)ₐ, a étant un nombre entier compris entre 1 et 6, par exemple le nombre 1, 2, 3 ou 4 ou un radical naphtyle,
- le radical alkyloxy est de préférence un radical méthoxy, éthoxy, propyloxy, isopropyloxy, n-butyloxy, isobutyloxy, tert-butyloxy, n-pentyloxy, isopentyloxy, sec-pentyloxy, tert-pentyloxy, néopentyloxy, n-hexyloxy, sec-hexyloxy, tert-hexyloxy,
- le radical alkylthio correspondant peut être utilisé en reprenant les mêmes valeurs et en remplaçant l'atome d'oxygène par un atome de soufre, exemple : méthylthio, éthylthio .... De plus, l'atome de soufre peut être oxydé, exemple : méthylsulfinyle, méthylsulfonyle .... le radical alkényloxy est de préférence un radical vinyloxy, 1-propényloxy, allyloxy, 1-butényloxy, 2-butényloxy, 3-butényloxy, 2-méthyl-1-butényloxy, pentényloxy, hexényloxy, 3-méthyl 2-butényloxy,
- le radical alkénylthio correspondant peut être utilisé en reprenant les mêmes valeurs et en remplaçant l'oxygène par un soufre éventuellement oxydé,
- le radical alkynyloxy est de préférence un radical éthynyloxy, propargyloxy, propynyloxy, butynyloxy, pentynyloxy, hexynyloxy,
- le radical alkynylthio correspondant peut être utilisé en reprenant les mêmes valeurs et en remplaçant l'oxygène par un soufre éventuellement oxydé,
- le radical aryloxy est de préférence un radical phényloxy, thiényloxy, furyloxy, thiazolyloxy, thiadiazolyloxy, oxazolyloxy, tétrazolyloxy, pyrrolyloxy, imidazolyloxy, pyrazolyloxy, isothiazolyloxy, isoxazolyloxy, triazolyloxy, thiatriazolyloxy, pyridyloxy, ainsi que les groupements condensés tels que benzothiényloxy, benzofuryloxy, indolyloxy, benzimidazolyloxy.
- Les groupements arylthio éventuellement oxydés correspondants peuvent bien entendu être utilisés, par exemple : phénylthio, phénylsulfonyle, phénylsulfinyle ...
- le radical aralkyloxy est de préférence un radical benzyloxy, phényléthyloxy, phénylpropyloxy, thiénylméthyloxy, thiényléthyloxy, thiénylpropyloxy, furfuryloxy, furyléthyloxy, furylpropyloxy, thiazolylméthyloxy, thiazolyléthyloxy, tétrazolylméthyloxy, thiadiazolylméthyloxy, thiadiazolyléthyloxy,
- Les groupements aralkylthio éventuellement oxydés correspondants peuvent bien entendu être utilisés.

Parmi les radicaux formyle protégés, on peut citer plus spécialement les radicaux du type acétal. On préfère les radicaux suivants : 1,3-dioxolan-2-yle, diméthoxyméthyle, diéthoxyméthyle.

Comme radicaux carboxyle estérifiés, on peut citer les radicaux alcoxycarbonyle ayant au plus 7 atomes de carbone tels que méthoxycarbonyle, éthoxycarbonyle, propyloxycarbonyle, isopropyloxycarbonyle, butyloxycarbonyle.

On peut également citer les radicaux alkyloxyalkyloxycarbonyle tels que méthoxyméthoxycarbonyle, isopropyloxyméthoxycarbonyle, les radicaux alkylthiométhoxycarbonyle tels que méthylthiométhoxycarbonyle, isopropylthiométhoxycarbonyle, les radicaux acyloxyalkyloxycarbonyle tel que pivaloyloxyméthoxycarbonyle, acétoxyéthoxycarbonyle.

Parmi les sels formés avec le groupement carboxyle, on peut citer les sels de sodium, potassium, lithium, calcium, magnésium, ammonium ou les sels formés avec les bases organiques aminées telles que la triméthylamine, la diéthylamine, la triéthylamine, le tris(hydroxyméthyl) aminométhane.

Parmi les radicaux acyle, on peut citer notamment les radicaux acétyle, propionyle, butyryle, isobutyryle, n-valéryle, isovaléryle, tert-valéryle et pivalyle.

Parmi les composés préférés de l'invention, on peut citer ceux dans lesquels Z représente un atome d'hydrogène, ceux dans lesquels R₁ représente un radical NH(CH₂)ₙAr, n et Ar conservant leur signification précédente, et notamment ceux dans lesquels Ar représente un radical : 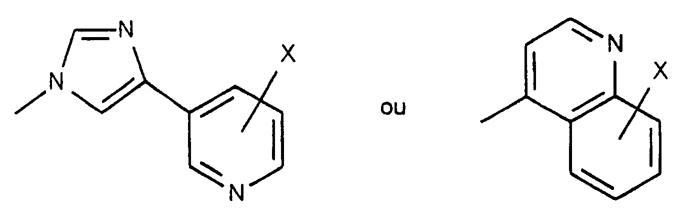 et dans lesquels X représente un atome d'hydrogène, un radical CH₃, OH, OMe, ou un atome d'halogène, tout spécialement ceux dans lesquels Ar représence un radical : 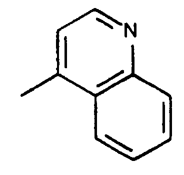 et ainsi que ceux dans lesquels n représente le nombre 3.

Parmi les composés préférés, on peut citer également les composés de formule (I), dans lesquels R représente un atome d'hydrogène, un radical : 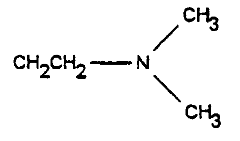 ou un radical : 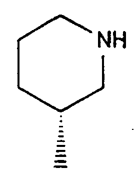 éventuellement substitué sur l'atome d'azote par un radical 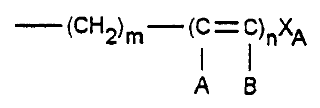 dans lequel m représente un nombre entier compris entre 0 et 20, n représente le nombre 0, 1, 2, ou 3,
et ou bien A et B identiques ou différents représentent un atome d'hydrogène ou un atome d'halogène ou un radical alkyle ou aryle, renfermant jusqu'à 8 atomes de carbone, la géométrie de la double liaison étant E ou Z ou un mélange E + Z, ou bien A et B forment une troisième liaison entre les atomes de carbone auxquels ils sont liés,
et X_{A} représente :
. un radical alkyle linéaire ou ramifié, saturé ou insaturé renfermant de 6 à 20 atomes de carbone, éventuellement interrompu par un ou plusieurs hétéroatomes et éventuellement substitué par un ou plusieurs atomes d'halogène,
. un radical alkyle cyclique renfermant de 3 à 8 atomes de carbone éventuellement substitué par un radical aryle carbocyclique,
. un atome d'halogène,
. un radical C≡N,
. un radical OR₃, COR₄, CO₂R₅, SR₆, 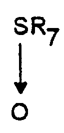 SO₂R₈ ou 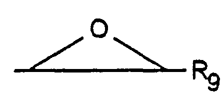 dans lequel R₃, R₄, R₅, R₆, R₇, R₈ et R₉ représentent un atome d'hydrogène, un radical alkyle renfermant jusqu'à 8 atomes de carbone, éventuellement interrompu par un ou plusieurs hétéroatomes et éventuellement substitué par un ou plusieurs atomes d'halogène, un radical aryle ou aralkyle, carbocyclique ou hétérocyclique renfermant jusqu'à 14 atomes de carbone, éventuellement substitué par un ou plusieurs radicaux choisis dans le groupe constitué par les radicaux carboxyle libre, salifié, estérifié ou amidifié, les radicaux hydroxyles, les atomes d'halogène, les radicaux NO₂, les radicaux C≡N, les radicaux alkyle, alkényle et alkynyle, O-alkyle, O-alkényle et O-alkynyle, S-alkyle, S-alkényle et S-alkynyle, SO-alkyle, SO-alkényle, SO-alkynyle, SO₂-alkyle, SO₂-alkényle, SO₂-alkynyle, renfermant jusqu'à 12 atomes de carbone éventuellement substitués par un ou plusieurs atomes d'halogène, les radicaux aryle, O-aryle, S-aryle carbocycliques ou hétérocycliques renfermant jusqu'à 14 atomes de carbone, les radicaux : 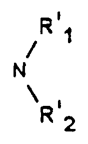 R'₁ et R'₂ identiques ou différents représentant un atome d'hydrogène ou un radical alkyle, renfermant jusqu'à 12 atomes de carbone, les radicaux aryles eux-mêmes éventuellement substitués par l'un des substituants indiqués ci-dessus comme substituants des radicaux aryles,
. un radical NR₁R₂ dans lequel ou bien R₁ et R₂ identiques ou différents représentent un atome d'hydrogène ou un radical alkyle renfermant jusqu'à 20 atomes de carbone, -CO-alkyle ou -CO₂-alkyle renfermant jusqu'à 8 atomes de carbone, ou un radical aryle, -CO-aryle ou -CO₂-aryle aralkyle, -CO-aralkyle ou -CO₂-aralkyle renfermant jusqu'à 14 atomes de carbone, les radicaux aryles étant eux-mêmes éventuellement substitués par l'un des substituants indiqués ci-dessus comme substituants des radicaux aryles, ou bien R₁ et R₂ forment ensemble avec l'atome d'azote auquel ils sont liés un cycle de 3 à 8 chaînons comportant éventuellement un autre hétéroatome et éventuellement substitué par l'un des substituants indiqués ci-dessus comme substituants des radicaux aryles,
. un radical aryle carbocyclique éventuellement substitué par un ou plusieurs des radicaux énoncés ci-dessus comme substituants des radicaux aryles,
. un radical aryle hétérocyclique comportant un ou plusieurs hétéroatomes, éventuellement substitué par un ou plusieurs des radicaux mentionnés ci-dessus comme substituants des radicaux aryles,
. un radical OC(Ar)₃ dans lequel Ar représente un radical aryle carbocyclique éventuellement substitué par un ou plusieurs des substituants mentionnés ci-dessus comme substituants des radicaux aryle.

L'invention a tout spécialement pour objet les composés dont la préparation est donnée ci-après dans la partie expérimentale et tout particulièrement le produit de l'exemple 3.

Les produits de formule générale (I) possèdent une très bonne activité antibiotique sur les bactéries gram^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, broncho-pneumonies, suppuration pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma, ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, le produit de l'exemple 3 et ses sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

L'invention a également pour objet un procédé de préparation caractérisé en ce que l'on soumet un composé de formule (II) : 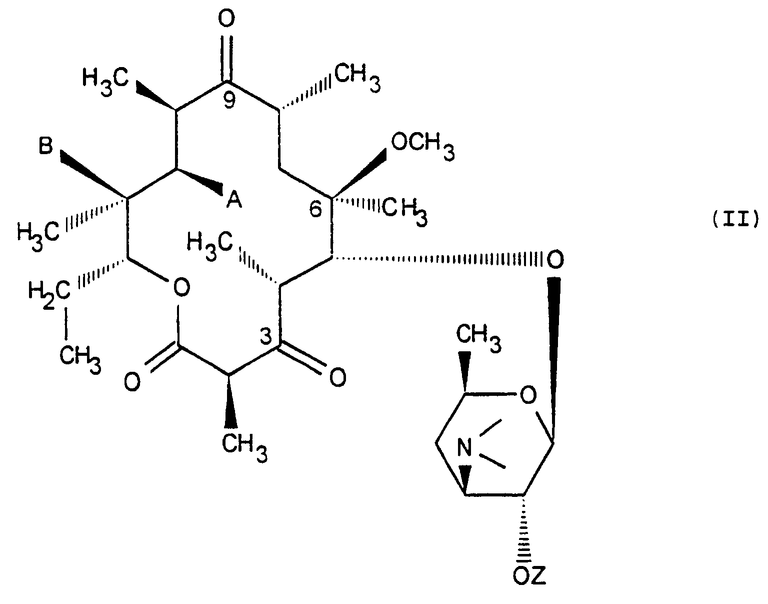 dans lequel A, B et Z conservent leur signification précédente à l'action d'un composé de formule (III) :

HN₂OR

dans lequel R conserve sa signification précédente pour obtenir le composé de formule (I) correspondant, puis si désiré, libère l'hydroxyle en 2' et/ou soumet à l'action d'un acide pour en former le sel, et/ou à l'action d'un agent de modification du radical R.

Les composés de formule (I) utilisés comme produit de départ peuvent être préparés selon le procédé décrit dans le brevet européen 0676409. Dans un mode de réalisation préféré:
- la libération de l'hydroxyle en 2' est effectuée par méthanolyse,
- la salification est réalisée selon les procédés classiques.

La 11,12-didéoxy-3-de((2,6-didéoxy-3-C-méthyl-3-O-méthyl-alpha-L-ribohexopyranosyl)oxy)-6-O-méthyl-3-oxo-12,11-(oxycarbonyl(2-(3-(4-quinoléinyl)propyl)hydrazono)) érythromycine est appelée ci-après produit A, le produit est décrit à l'exemple 5 du brevet EP 0676409.

### EXEMPLE 1 : (E) 9-O-(phénylméthyl) oxime de 11,12-didéoxy-3-de((2,6-didéoxy-3-C-méthyl-3-O-méthyl-alpha-L-ribohexopyranosyl)oxy)-6-O-méthyl-3-oxo-12,11-(oxycarbonyl(2-(3-(4-quinoléinyl)propyl)hydrazono))-érythromycine

On agite au reflux pendant 10 jours un mélange de 1 g de produit A, 25 cm³ d'éthanol de 0,91 g de chlorhydrate de benzylhydroxylamine. On extrait à l'acétate d'éthyle, lave avec une solution d'ammoniaque à 10 %, sèche sur sulfate de magnésium et évapore à sec. On chromatographie le produit obtenu en éluant avec le mélange chlorure de méthylène, méthanol, ammoniaque 97-3-0,4. On obtient 0,69 g de produit que l'on purifie par chromatographie sur silice en éluant avec le mélange chlorure de méthylène, alcool isopropylique ammoniaque (94-6-0,4). On obtient 120 mg de produit que l'on reprend dans l'éther éthylique, filtre le précipité, évapore à sec, reprend dans l'acétate d'éthyle, lave à l'ammoniaque, sèche sur sulfate de magnésium et évapore à sec. On obtient 90 mg de produit recherché.
RMN CDCl₃ ppm
0,78 (t) : CH₃-CH₂ ; 0,98 (d) : 8-Me ; 1,09 (d) : 10-Me ; 1,27 (d) : 5'-Me ; 1,31 (d) : 2-Me ; 1,43 et 1,46 : 6 et 12-Me ; ∼ 1,25 et 1,69 : CH₂ en 4' ; ∼ 1,40 et 1,69 : CH₂ en 7 ; ~ 1,55 et 1,95 : CH₂ central chaîne ; 2,29 (s) : N(Me)₂ ; ∼ 2,33 et 2,80 (m) - 5,84 (t) : CH₂NH ; 2,48 (m) : H'₃ ; 2,66 (q) : H₁₀ ; 2,70 (s) : 6-OMe ; 3,09 (m) : H₅ ; 3,20 (dd) : H'₂ ; 3,10 à 3,30 : CH₂C= ; 3,55 (m) : H'₅ ; 3,78 (m) : H₈ --> isomère E ; 3,87 (s) : H₁₁ ; 3,88 (q) : H₂ ; 4,29 : H'₁ et H₅ ; 4,87 (d) et 4,98 (d) : OCH₂Φ ; 5,02 (dd) : H₁₃ ; 7,15 à 7,30 : phényle et H₃ quinoléine ; 7,51 et 7,67 : H₆ et H₇ - 8,09 et 8,13 : H₅ et H₈ : quinoléine ; 8,78 (d) : H₂.

### EXEMPLE 2 : 9(E) 9-oxime de 11,12-didéoxy-3-de[(2,6-didéoxy-3-C-méthyl-3-O-méthyl-alpha-L-ribohexopyranosyl)oxy]-6-O-méthyl-3-oxo-12,11-[oxycarbonyl[[3-(4-quinoléinyl)propyl] hydrazono]]-érythromycine

On agite pendant 6 semaines à 105°C un mélange de 1 g du produit A, 25 cm³ d'éthanol et 311 mg de chlorhydrate d'hydroxylamine. On chromatographie sur silice en éluant avec le mélange chlorure de méthylène, méthanol, ammoniaque 92-8-0,6. On évapore à sec le produit obtenu, reprend dans l'acétate d'éthyle, lave avec une solution d'ammoniaque à 5 %, sèche et évapore à sec. On obtient 1,029 g de produit que l'on purifie par chromatographie sur silice en éluant avec le mélange chlorure de méthylène, méthanol, ammoniaque 96-4-0,4. On obtient 430 mg de produit que l'on purifie sur silice en éluant avec le mélange chlorure de méthylène-méthanol-ammoniaque (96-4-0,4). On obtient 272 mg de produit recherché.
RMN CDCl₃ ppm
0,86 (t) : CH₃-CH₂ ; 1,06 (dl) - 1,19 (d) - 1,29 (d) - 1,33 (d) - 1,38 (d) : les CH₃-CH ; 1,51 (s) - 1,60 (s) 6 et 12-Me ; 2,28 (S) : N(Me)₂ ; 2,47 (m) : H'₃ ; 2,73 (ql) : H₁₀ ; 2,84 (m) : CH₂-NH ; 2,89 (s) : 6-OMe ; 2,90 à 3,15 : CH₂-O= ; ~ 3,14 (m) : H₄ ; 3,24 (dd) : H'₂ ; 3,58 (m) : H'₅ ; 3,92 (s) : H₂ ; 3,95 (s) : H₁₁ ; -3,95 (m) : H₈ --> isomère E ; -4,31 : H'₁ et H₅ ; 5,09 (dd) : H₁₃ ; 6,75 (d) : H₃ ; 8,35 (d) : H₂ ; 7,43 et 7,61 : H₆ et H₇ - 7,89 et 7,99 : H₅ et H₈ : quinoléine ; ∼6,20 et 11,45 : OH oxime et NH ; + éther ∼ 1/3 de mole.

### EXEMPLE 3 : (9E) 9-[O-(3-pipéridinyl) de 11,12-didéoxy-3-de[(2,6-didéoxy-3-C-méthyl-3-O-méthyl-alpha-L-ribohexopyranosyl)oxy]-6-O-méthyl-3-oxo-12,11-[oxycarbonyl[[3-(4-quinoléinyl)propyl] hydrazono]]-érythromycine

On ajoute 8 cm³ d'une solution d'acide chlorhydrique dans l'éthanol 0,5 N dans une solution renfermant 1,12 g de N-méthylbenzyle-3-hydroxylamine pipéridine et 5 cm³ d'éthanol. On ajoute à la solution obtenue, 1 g de produit A en solution dans 10 cm³ d'éthanol. On agite au reflux pendant 4 jours. On ajoute à nouveau 0,76 g d'hydroxylamine en solution dans 5 cm³ d'éthanol, amène à pH ∼ 3,5 par ajout d'une solution d'acide chlorhydrique et d'éthanol. On évapore à sec, reprend dans l'acétate d'éthyle, lave à l'ammoniaque, sèche et évapore à sec. On purifie en éluant avec le mélange acétate d'éthyle, triéthylamine 95-5. On obtient 585 mg de produit.

On dissout 357 mg de ce produit B dans une solution renfermant 10 cm³ de méthanol et 53 mg de palladium sur charbon. On agite sous atmosphère d'hydrogène pendant 5 heures. On filtre, rince au méthanol et évapore à sec. On chromatographie sur silice en éluant avec le mélange chlorure de méthylène-méthanol-ammoniaque (96-4-1). On obtient 150 mg de produit.
RMN CDCl₃ ppm
=N-O-CH=, on localise ; 0,73 (t) - 0,76 (t) : CH₃-CH₂ ; 1,00 (d) : 8-Me ; 1,14 (d) : 10-Me ; 1,26 (d) : 5'-Me ; 1,30 (d) : 4-Me ; 1,36 (d) : 2-Me ; 1,42 - 1,47 : 6 et 12-Me ; 2,27 (s) N(Me)₂ ; 2.69 - 2,71 : 6-OMe ; 2,45 (m) : H'₅ ; ~ 2,71 (masqué) : H₁₀ ; 3,08 (m) : H₄ : 3,19 (dd) : H'₂ ; ~ 2,79 (m) : CH₂NHNC ; 2,65 à 3,35 : les CH₂N et CH₂Φ ; 3,55 (m) : H'₅ ; 3,74 (m) : H₈ --> DE, ne semble pas déficitaire ; 3,82-3,98 : O-CH= ; 3,87 (s) : H₁₁ : 5,03 (dd) : H₁₃ ; 3,89 (q) : H₂ ; 6,06-6,10 : NH-NC=O ; ~ 7,31 : H₃ ; 8,77 : H₂ - 7,52 et 7,66 : H₆ et H₇ - ~ 8,09 et 8,12 : H₅ et H₈ : quinoléine.

### EXEMPLE 4 : (9E) 9-[O-(4-pipéridinyl) oxime] de 11,12-didéoxy-3-de[(2,6-didéoxy-3-C-méthyl-3-O-méthyl-alpha-L-ribohexopyranosyl)oxy]-6-O-méthyl-3-oxo-12,11-[oxycarbonyl [[3-(4-quinoléinyl)propyl] hydrazono]]-érythromycine

On agite au reflux pendant 9 jours une solution renfermant 311 mg du produit A, 10 cm³ d'éthanol, 400 mg de N-méthylbenzyle 4-hydroxylamine pipéridine. On évapore à sec, extrait à l'acétate d'éthyle, lave avec une solution aqueuse d'ammoniaque à 5 %, sèche sur sulfate de magnésium et évapore à sec. On obtient 760 mg de produit que l'on chromatographie sur silice en éluant avec le mélange acétate d'éthyle-triéthylamine (98-2). On obtient 170 mg de produit. On agite sous atmosphère d'hydrogène, pendant 4 heures, un mélange de 105 mg de ce produit, 6 cm³ de méthanol, 15 mg de palladium sur charbon. On filtre, rince et évapore à sec. On obtient 69 mg de produit recherché après purification.

### EXEMPLE 5 : (E) 9-(O-2-bromoéthyl) oxime de 11,12-didéoxy-3-de[2,6-didéoxy-3-C-méthyl-3-O-méthyl-alpha-L-ribohexopyranosyl-oxy]-6-O-méthyl-3-oxo-12,11-[oxycarbonyl [[3-(4-quinoléinyl)propyl]hydrazono]]-érythromycine

On agite pendant 14 jours au reflux une solution, renfermant 303 mg du produit A et 1000 mg de Br(CH₂)₂ONH₂, HBr. On évapore à sec, reprend dans l'acétate d'éthyle, lave avec une solution d'ammoniaque à 5 %, sèche sur sulfate de magnésium et évapore à sec. On obtient 550 mg de produit que l'on chromatographie sur silice en éluant avec le mélange acétate d'éthyle triéthylamine (98-2). On obtient 60 mg de produit recherché.
RMN CDCl₃ ppm
0,79 (t) : CH₃-CH₂ ; 1,02 (d) - 1,13 (d) - 1,2 (dd) - 1,30 (d) - 1,36 (d) - 1,30 (d) - 1,36 (d) - 1,43 - 1,48 : 6 et 12-Me ; 2,27 (s) : N(Me)₂ ; 2,45 (m) : H'₃ ; 2,70 (s) : 6-OMe ; ∼ 3,08 (m) : H₄ ; 3,19 (dd) : H'₂ ; ~ 3,19 (dd) : H'₂ ; ∼ 3,27 (m) : H₁₀ ; 2,4 à 3,0 - 3,27 (m) : CH₂-C= et CH₂NH ; 3,45 (t) : CH₂Br ; 3,54 (m) : H'₅ ; 3,73 (m) : H₈ --> isomère E ; 3,88 (s) : H₁₁ ; 3,88 (q) : H₂ ; 4,1 à 4,3 : CH₂ON= ; 4,29 : H'₁ et H₅ ; 5,03 (dd) : H₁₃ ; 5,88 (t) : NHCH₂ ; 7,29 (d) : H₃ ; 7,53 et 7,68 : H₆ et H₇ - 8,09 et 8,13 : H₅ et H₈ - 8,78 : H₂ : quinoléine.

### EXEMPLE 6 : (E) 9-[O-[2-[(4-phénylbutyl) amino] éthyl] oxime de 11,12-didéoxy-3-de[(2,6-didéoxy-3-C-méthyl-3-O-méthyl-alpha-L-ribohexopyranosyl)-oxy]-6-O-méthyl-3-oxo-12,11-[oxycarbonyl [[3-(4-quinoléinyl) propyl] hydrazono]] érythromycine

On agite pendant 2 jours à la température ambiante un mélange de 144 mg du produit préparé à l'exemple précédent et 1,41 g de 4-phényl butylamine. On chromatographie sur silice le produit obtenu en éluant avec le mélange chlorure de méthylène-méthanol-ammoniaque (96-4-1), puis on purifie une seconde fois avec le mélange acétate d'éthyle triéthylamine (95-5). On obtient 66 mg du produit.
RMN CDCl₃ ppm
0,78 (t) : CH₃-CH₂ ; 0,99 (d) : CH₃ en 8 ; 1,12 (d) : CH₃ en 10 ; 1,24 (d) : CH₃ en 5' ; 1,30 (d) : CH₃ en 4 ; 1,36 (d) CH₃ en 2 ; 1,40 et 1,46 (s) : CH₃ en 6 et 12 ; 2,27 (s) : N(CH₃)₂ ; 2,69 (s) : CH₃O en 6 ; 2,46 (m) : H₃' ; ~ 1,20 (m) ~ 1,70 (m) : CH₂ en 4' ; ~ 1,40 (m) ~ 1,70 (m) : CH₂ en 7 ; 1,60 (m) ∼ 1,95 (m) : CH₂ en 14 ; 1,50 à 2,10 (m) : CH₂ centraux des chaînes ; 2,61 (m) ; 4H - 2,70 à 3,30 (m) : les N-CH₂ et CH₂-Ar ; ~ 2,69 (masqué) : H₁₀ ; 3,07 (m) : H₄ ; 3,19 (dd, J = 10 et 7,5) : H₂' ; 3,54 (m) : H₅' : 3,67 (m) : H₈ ; 3,86 (s) : H₁₁ ; 3,87 (q) : H₂ ; 3,92 à 4,08 (m) : O-CH2-CH₂ ; 4,27 (m) : H₁' + H₅ ; 5,02 (dd) : H₁₃ ; 5,99 (t) : 1H mobile.

### EXEMPLE 7 : (E) 9-[O-[2-(diméthylamino) éthyl] oxime] de 11,12-didéoxy-3-de[(2,6-didéoxy-3-C-méthyl-3-O-méthyl-alpha-L-ribohexopyranosyl)-oxy]-6-O-méthyl-3-oxo-12,11-[oxycarbonyl [[3-(4-quinoléinyl) propyl] hydrazono]] érythromycine

On agite pendant 1 heure à 75°C, 250 mg de produit obtenu à l'exemple 5 en solution dans 10 cm³ de diméthylamine dant EtOH à 33 %. On chromatographie sur silice en éluant avec le mélange chlorure de méthylène-méthanol-ammoniaque (96-4-1). On obtient 210 mg de produit que l'on reprend dans l'acétate d'éthyle, lave avec une solution aqueuse d'ammoniaque à 5 %, sèche sur sulfate de magnésium et évapore à sec. On obtient 190 mg de produit recherché.
RMN CDCl₃ ppm
0,78 (t) : CH₃-CH₂ ; 3,07 (m) : H₄ ; 3,88 (q) : H₂.

### EXEMPLE 8 :

En opérant comme précédemment on a préparé la 9-méthyloxime du composé A, à partir du composé A et de CH₃ONH₂.

### EXEMPLE 9 : (E) 9- [O-[2-[4-(3-pyridinyl)-1H-imidazol-1-yl] éthyl] oxime] de 11,12-didéoxy-3-de[(2,6-didéoxy-3-C-méthyl-3-O-méthyl-alpha-L-ribo-hexopyranosyl) oxy] 6-O-méthyl-3-oxo-12,12-[oxycarbonyl-[[3-(4-quinoleinyl) propyl] hydrazono]] érythromycine

Ce produit a été préparé à partir du produit de l'exemple 5 en opérant comme à l'exemple 6 en utilisant du pyridinoimidazole.

### EXEMPLE 10 : (E) 9-[O-3-piperidinyl] de 11,12-didéoxy-3-de[(2,6-didéoxy-3-C-méthyl-3-O-méthyl-alpha-L-ribo-hexopyranosyl) oxy] 6-O-méthyl-3-oxo-12,11-[oxycarbonyl-(hydrazono)] érythromycine.

### Stade A : (E) 9-[O-[1-[(phénylméthoxy) carbonyl] 3-pipéridinyl] oxime] de 11,12-didéoxy-3-de[(2,6-didéoxy-3-C-méthyl-3-O-méthyl-alpha-L-ribo-hexopyranosyl) oxy] 6-O-méthyl-3-oxo-12,11-[oxycarbonyl-(hydrazono)] érythromycine.

On mélange à température ambiante 1,19 g de N-méthyl benzyle 3-hydroxylamine pipéridine et 1 g de 11,12-didéoxy-3-de[(2,6-didéoxy-3-C-méthyl-3-O-méthyl-alpha-L-ribo-hexopyranosyl) oxy] 6-O-méthyl-3-oxo-12,11-[oxycarbonyl-(hydrazono)] érythromycine obtenue comme indiqué dans la demande de brevet européen EP 0676409 dans 10 ml d'éthanol. On chauffe à 105°C pendant 6 jours puis élimine le solvant sous pression réduite, reprend le résidu dans l'acétate d'éthyle, lave à l'eau, sèche, et évapore le solvant. On obtient après purification par chromatographie sur silice (éluant : CH₂Cl₂-MeOH-NH₄OH 96-4-0,5), 1 g de produit attendu.

### Stade B : (E) 9-[O-3-piperidinyl] de 11,12-didéoxy-3-de[(2,6-didéoxy-3-C-méthyl-3-O-méthyl-alpha-L-ribo-hexopyranosyl) oxy] 6-O-méthyl-3-oxo-12,11-[oxycarbonyl-(hydrazono)] érythromycine.

On mélange à température ambiante 250 mg de produit obtenu au stade A dans 10 ml de dichlorométhane en présence de 150 mg de palladium sur charbon actif. On hydrogène et agite à température ambiante pendant 24 heures. On filtre, rince au dichlorométhane, évapore le solvant sous pression réduite, chromatographie le résidu sur silice (éluant : CH₂Cl₂-MeOH-NH₄OH 92-8-0,5), obtient 166 mg de produit pur que l'on reprend dans l'acétate d'éthyle, lave avec de l'ammoniaque diluée, sèche, évapore le solvant et obtient 130 mg de produit attendu.
RMN CDCl₃ ppm
0,85 : CH₃-CH₂ ; 1,00 : 8-Me ; 1,16 : 10-Me ; ≃ 1,27 : 5'Me et 4Me ; 1,35 : 2-Me ; 1,27 et 1,45 : 6-Me et 12-Me ; 2,27 : NMe₂ ; 2,48 : H₃' ; 2,68 : H₁₀ ; 2,70 : 6-OMe ; ≃ 2,70-2,83-3,08 : les CH₂-NH ; 3,08 : H₄ ; 3,19 : H₂' ; 3,55 : H₅' ; 3,72 : H₁₁ ; ≃ 3,72 (m) : H₈ → isomère E ; 3,85 : H₂ ; 3,92 : 0-CH< ; 4,24 : H₅ ; 4,31 : H₁' ; 5,06 : H₁₃.

### EXEMPLE 11 : (E) 9-[O-3-piperidinyloxime] de 11,12-didéoxy-3-de[(2,6-didéoxy-3-C-méthyl-3-O-méthyl-alpha-L-ribo-hexopyranosyl) oxy] 6-O-méthyl-3-oxo-12,11-[oxycarbonyl-(propylhydrazono)] érythromycine.

### Stade A : (E) 9-[O-[1-[(phénylméthoxy) carbonyl] 3-pipéridinyl] oxime] de 11,12-didéoxy-3-de[(2,6-didéoxy-3-C-méthyl-3-O-méthyl-alpha-L-ribo-hexopyranosyl) oxy] 6-O-méthyl-3-oxo-12,11-[oxycarbonyl-(propylhydrazono)] érythromycine.

On agite pendant 24 heures à température ambiante 250 mg de produit obtenu au stade A de l'exemple 10 dans 5 ml de méthanol en présence de 50 µl de propanaldéhyde et 52 mg d'acide acétique glacial. On ajoute ensuite 55 mg de cyanoborohydrure de sodium, agite 3 jours à température ambiante, ajoute de l'acétate d'éthyle, lave avec une solution aqueuse de soude N puis à l'eau, sèche, évapore le solvant sous pression réduite, chromatographie le résidu sur silice (éluant : CH₂Cl₂-MeOH-NH₄OH 96-4-0,5) et obtient 262 mg de produit attendu.

### Stade B : (E) 9-[O-3-piperidinyloxime] de 11,12-didéoxy-3-de[(2,6-didéoxy-3-C-méthyl-3-O-méthyl-alpha-L-ribo-hexopyranosyl) oxy] 6-O-méthyl-3-oxo-12,11-[oxycarbonyl-(propylhydrazono)] érythromycine.

On opère comme au stade B de l'exemple 10 en utilisant au départ 262 mg du produit obtenu au stade A précédent, 150 mg de palladium sur charbon actif dans 10 ml de dichlorométhane. Après purification, on obtient 136 mg de produit attendu.
RMN CDCl₃ ppm
0,86 : H₁₅ ; 0,97 : CH₃ ; 1,00 : 8Me ; 1,13 : 10Me ; 1,26 : 5'Me ; 1,3 : 4Me ; 1,36 : 2Me ; 1,39 : H₇ ; 1,42-1,47 : 6Me et 12Me ; 1,53 : CH₂ ; 1,63 : 4" ; 1,64-1,46 : 5" ; 1,68-1,23 : 4' ; 1,97-1,56 : H₁₄ ; 2,28 : NMe ; 2,47 : 3' ; 2,66 : H₁₀ ; 2,68 : CH₂ ; 2,7-2,83 : 6" ; 2,72 : 60Me ; 3,07-2,78 : 2" ; 3,09 : H₄ ; 3,19 : 2' ; 3,55 : 5' ; 3,74 : H₈ ; 3,87 : H₁₁ ; 3,88 : H₂ ; 3,9 : 3" ; 4,27 : H₅ ; 4,29 : 1' ; 5,06 : H₁₃ ; 5,87 : NH.

### EXEMPLE 12 : (E) 9-[O-3-piperidinyloxime] de 11,12-didéoxy-3-de[(2,6-didéoxy-3-C-méthyl-3-O-méthyl-alpha-L-ribo-hexopyranosyl) oxy] 6-O-méthyl-3-oxo-12,11-[oxycarbonyl-[(3-phénylpropyl) hydrazono)]] érythromycine.

### Stade A : (E) 9-[O-[1-[(phénylméthoxy) carbonyl] 3-pipéridinyl] oxime] de 11,12-didéoxy-3-de[(2,6-didéoxy-3-C-méthyl-3-O-méthyl-alpha-L-ribo-hexopyranosyl) oxy] 6-O-méthyl-3-oxo-12,11-[oxycarbonyl-[(3-phénylpropyl) hydrazono)]] érythromycine.

On opère comme au stade A de l'exemple 11 en utilisant au départ 311 mg du produit obenu au stade A de l'exemple 10, 99 mg de phénylpropanaldéhyde, 66 mg d'acide acétique dans 10 ml de méthanol puis 69 mg de cyanoborohydrure de sodium. Après chromatographie, on obtient 270 mg de produit attendu.

### Stade B : (E) 9-[O-3-piperidinyloxime] de 11,12-didéoxy-3-de[(2,6-didéoxy-3-C-méthyl-3-O-méthyl-alpha-L-ribo-hexopyranosyl) oxy] 6-O-méthyl-3-oxo-12,11-[oxycarbonyl-[(3-phénylpropyl) hydrazono)]] érythromycine.

On opère comme au stade B de l'exemple 10 en utilisant au départ 270 mg du produit du stade A ci-dessus, 100 mg de palladium sur charbon actif et 10 ml de méthanol. Après purification, on obtient 83 mg de produit attendu.
RMN CDCl₃ ppm
0,85 : CH₃-CH₂ ; 1,00 : 8Me ; 1,12 : 10Me ; 1,22-1,68 : CH₂ en 4' ; 1,26 : 5'Me ; 1,3 : 4Me ; 1,36 : 2Me ; 1,38-1,7 : CH₂ en 7 ; 1,39 : H₇ ; 1,41-1,47 : 6Me et 12Me ; 1,5 à 2 : CH₂C cycl. ; 1,57-1,95 : CH₂CH₃ ; 1,83 : CH₂ central ; 2,28 : NMe2; 2,45 : 3' ; 2,67 à 2,87 : H₁₀ et 6-OMe ; 2,68 : CH₂ ; 3,1 : H₄ ; 3,18 : 2' ; 3,55 : 5' ; 3,73 : H₈ ; 3,86 : CH-OH ; 3,87 : H₁₁ ; 3,88 : H₂ ; 3,9 : 3" ; 4,27 : H₅ ; 4,29 : 1' ; 5,07 : H₁₃ ; 7,15 à 7,25 : phényle.

### EXEMPLES DE COMPOSITIONS PHARMACEUTIQUES

On a préparé des composés renfermant :

| | |
|---|---|
| Produit de l'exemple 3 | 150 mg |
| Excipient q.s.p. | 1 g |

Détail de l'excipient : amidon, talc, stéarate de magnésium.

### ETUDE PHARMACOLOGIQUE DES PRODUITS DE L'INVENTION

### Méthode des dilutions en milieu liquide

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination, ce qui permet de déterminer les concentrations minimales inhibitrices (C.M.I.) exprimées en microgrammes/cm³.

Les résultats suivants ont été obtenus :

| Souches bactériennes à GRAM⁺ | |
|---|---|
| Produits | Exemple 3 |
| Staphylococcus aureus 011UC4 | 0,3 |
| Streptococcus pyogenes groupe A 02A1UC1 | 0,08 |
| Streptococcus agalactiae groupe B 02B1HT1 | 0,08 |
| Streptococcus faecalis groupe D 02D2UC1 | 0,08 |
| Streptococcus faecium groupe D 02D3HT1 | 0,08 |
| Streptococcus mitis 02mitCB1 | 0,04 |
| Streptococcus agalactiae groupe B 02B1SJ1 | 0,08 |
| Streptococcus sanguis | 0,08 |
| Streptococcus pneumoniae 032UC1 | ≤ 0,02 |
| Streptococcus pneumoniae 030GR20 | 0,04 |
| Streptococcus pneumoniae 030PW23c | 0,08 |
| Streptococcus pneumoniae 030RO1i | 0,08 |
| Streptococcus pneumoniae 030SJ1c | 0,15 |

## Revendications

1. Les composés de formule (I) : 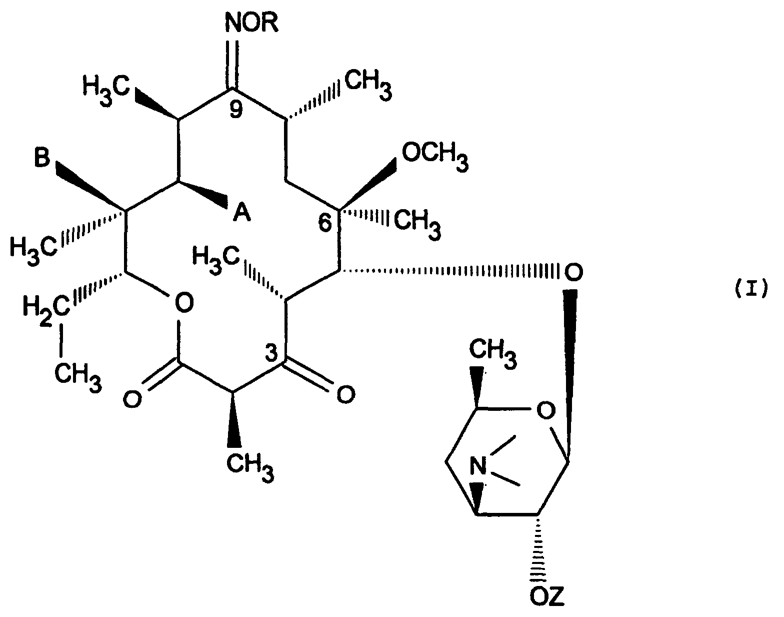 dans lesquels A et B forment avec les atomes auxquels ils sont liés un groupement : 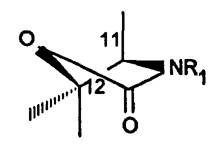
- R₁ représente un radical NH₂ ou un radical NH-alkyle, NH-alkényle ou NH-alkynyle renfermant jusqu'à 18 atomes de carbone ou un radical NH(CH₂)ₙAr ou N=CH(CH₂)ₙAr dans lequel n représente un nombre entier compris entre 1 et 6, et Ar représente un radical aryle ou hétéroaryle éventuellement substitué, Z représente un atome d'hydrogène ou le reste d'un acide carboxylique renfermant jusqu'à 18 atomes de carbone, et R représente :
- un atome d'hydrogène,
- un radical hétérocyclique renfermant au moins un atome d'azote et éventuellement un autre hétéroatome, mono ou bicyclique, saturé ou insaturé, aromatique ou non aromatique comportant jusqu'à 12 chaînons, éventuellement substitué sur l'atome d'azote,
- un radical alkyle, alkényle ou alkynyle linéaire, ramifié ou cyclique, renfermant jusqu'à 18 atomes de carbone éventuellement substitué par un ou plusieurs groupements :
. hydroxyle,
. halogène,
. cyano,
. nitro,
. amidinyle,
. guanidinyle,
. hétérocyclique, tel que défini précédemment,
. alkyloxy, alkényloxy ou alkynyloxy ayant au plus 6 atomes de carbone,
. alkylthio, alkénylthio ou alkynylthio ayant au plus 6 atomes de carbone, l'atome de soufre étant éventuellement oxydé en sulfoxyde ou en sulfone,
. aryle, aralkyle,
. aryloxy, aralkyloxy,
. arylthio, aralkylthio, l'atome de soufre étant éventuellement oxydé en sulfoxyde ou en sulfone,
. 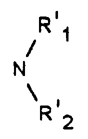 dans lequel :
**ou bien R'**_{**1**} **et R'**_{**2**} identiques ou différents représentent un atome d'hydrogène, un radical alkyle, alkényle ou alkynyle, linéaire, ramifié ou cyclique renfermant jusqu'à 18 atomes de carbone, un radical aryle ou aralkyle, chacun de ces radicaux R'₁ et R'₂ étant éventuellement substitué par un ou plusieurs radicaux hydroxy, alkyloxy, alkényloxy, alkynyloxy, alkylthio, alkénylthio ou alkynylthio renfermant jusqu'à 8 atomes de carbone, amino, monoalkylamino renfermant jusqu'à 4 atomes de carbone, dialkylamino renfermant jusqu'à 8 atomes de carbone, cyano, carboxyle libre, éstérifié ou salifié, acyle ou carbamoyle, renfermant jusqu'à 8 atomes de carbone, par un radical Si(alc)₃ ou Si(Oalc)₃ dans lequel alc représente un radical alkyle renfermant jusqu'à 4 atomes de carbone, par un radical hétérocyclique tel que défini précédemment,
**ou bien R'**_{**1**} **et R'**_{**2**} forment ensemble avec l'atome d'azote auquel ils sont liés un radical hétérocycle mono ou bicyclique, renfermant éventuellement un autre hétéroatome, saturé ou insaturé, aromatique ou non aromatique, comportant jusqu'à 12 chaînons ;
. un groupement ammonium quaternaire,
. 1,2-époxyéthyle ou 2,2-diméthyl 1,2-époxyéthyle ou un radical résultant de l'ouverture de ce groupement par un réactif nucléophile,
. 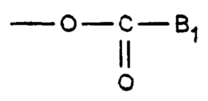 dans lequel B₁ représente soit un radical alkyle ou alkyloxy ayant au plus 6 atomes de carbone, soit un radical aryle, aralkyle, aryloxy ou aralkyloxy,
. formyle libre ou protégé, carboxyle libre, estérifié ou salifié, thiocyanate, acyle ou carbamoyle,
. (CH₂)ₙR', R' représentant le reste d'un acide aminé, et n représentant un nombre entier compris entre 0 et 6,
ainsi que les sels d'addition avec les acides des composés de formule (I).

2. Les composés de formule (I) définis à la revendication 1, dans lesquels Z représente un atome d'hydrogène.

3. Les composés de formule (I) définis à la revendication 1 ou 2, dans lesquels R₁ représente un radical NH(CH₂)ₙAr, n et Ar conservant leur signification indiquée à la revendication 1.

4. Les composés de formule (I) définis à l'une quelconque des revendications 1 à 4 dans lesquels Ar représente un radical : 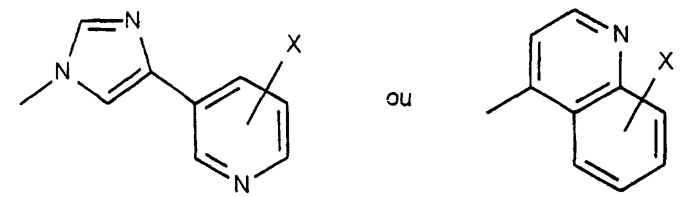 et dans lesquels X représente un atome d'hydrogène, un radical CH₃, OH, OMe, ou un atome d'halogène.

5. Les composés de formule (I) définis à la revendication 4 dans lesquels Ar représente un radical :

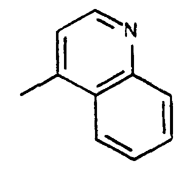

6. Les composés de formule (I) définis à la revendication 3, 4 ou 5 dans lesquels n représente le nombre 3.

7. Les composés de formule (I) définis à la revendication 1, dans lesquels R représente un atome d'hydrogène, un radical : 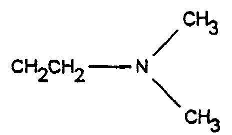 ou un radical : 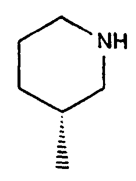 éventuellement substitué sur l'atome d'azote par un radical : 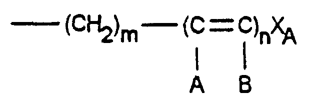 dans lequel m représente un nombre entier compris entre 0 et 20, n représente le nombre 0, 1, 2, ou 3,
et ou bien A et B identiques ou différents représentent un atome d'hydrogène ou un atome d'halogène ou un radical alkyle ou aryle, renfermant jusqu'à 8 atomes de carbone, la géométrie de la double liaison étant E ou Z ou un mélange E + Z, ou bien A et B forment une troisième liaison entre les atomes de carbone auxquels ils sont liés,
et X_{A} représente :
. un radical alkyle linéaire ou ramifié, saturé ou insaturé renfermant de 6 à 20 atomes de carbone, éventuellement interrompu par un ou plusieurs hétéroatomes et éventuellement substitué par un ou plusieurs atomes d'halogène,
. un radical alkyle cyclique renfermant de 3 à 8 atomes de carbone éventuellement substitué par un radical aryle carbocyclique,
. un atome d'halogène,
. un radical C≡N,
. un radical OR₃, COR₄, CO₂R₅, SR₆, 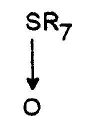 SO₂R₈ ou 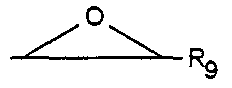 dans lequel R₃, R₄, R₅, R₆, R₇, R₈ et R₉ représentent un atome d'hydrogène, un radical alkyle renfermant jusqu'à 8 atomes de carbone, éventuellement interrompu par un ou plusieurs hétéroatomes et éventuellement substitué par un ou plusieurs atomes d'halogène, un radical aryle ou aralkyle, carbocyclique ou hétérocyclique renfermant jusqu'à 14 atomes de carbone, éventuellement substitué par un ou plusieurs radicaux choisis dans le groupe constitué par les radicaux carboxyle libre, salifié, estérifié ou amidifié, les radicaux hydroxyles, les atomes d'halogène, les radicaux NO₂, les radicaux C≡N, les radicaux alkyle, alkényle et alkynyle, O-alkyle, O-alkényle et O-alkynyle, S-alkyle, S-alkényle et S-alkynyle, SO-alkyle, SO-alkényle, SO-alkynyle, SO₂-alkyle, SO₂-alkényle, SO₂-alkynyle, renfermant jusqu'à 12 atomes de carbone éventuellement substitués par un ou plusieurs atomes d'halogène, les radicaux aryle, O-aryle, S-aryle carbocycliques ou hétérocycliques renfermant jusqu'à 14 atomes de carbone, les radicaux : 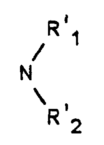 R'₁ et R'₂ identiques ou différents représentant un atome d'hydrogène ou un radical alkyle, renfermant jusqu'à 12 atomes de carbone, les radicaux aryles eux-mêmes éventuellement substitués par l'un des substituants indiqués ci-dessus comme substituants des radicaux aryles,
. un radical NR₁R₂ dans lequel ou bien R₁ et R₂ identiques ou différents représentent un atome d'hydrogène ou un radical alkyle renfermant jusqu'à 20 atomes de carbone, -CO-alkyle ou -CO₂-alkyle renfermant jusqu'à 8 atomes de carbone, ou un radical aryle, -CO-aryle ou -CO₂-aryle aralkyle, -CO-aralkyle ou -CO₂-aralkyle renfermant jusqu'à 14 atomes de carbone, les radicaux aryles étant eux-mêmes éventuellement substitués par l'un des substituants indiqués ci-dessus comme substituants des radicaux aryles, ou bien R₁ et R₂ forment ensemble avec l'atome d'azote auquel ils sont liés un cycle de 3 à 8 chaînons comportant éventuellement un autre hétéroatome et éventuellement substitué par l'un des substituants indiqués ci-dessus comme substituants des radicaux aryles,
. un radical aryle carbocyclique éventuellement substitué par un ou plusieurs des radicaux énoncés ci-dessus comme substituants des radicaux aryles,
. un radical aryle hétérocyclique comportant un ou plusieurs hétéroatomes, éventuellement substitué par un ou plusieurs des radicaux mentionnés ci-dessus comme substituants des radicaux aryles,
. un radical OC(Ar)₃ dans lequel Ar représente un radical aryle carbocyclique éventuellement substitué par un ou plusieurs des substituants mentionnés ci-dessus comme substituants des radicaux aryle.

8. Les composés de formule (I) définis à la revendication 1, dont le nom suit :
(9E) 9-[O-(3-pipéridinyl) oxime] de 11,12-didéoxy-3-de[(2,6-didéoxy-3-C-méthyl-3-O-méthyl-alpha-L-ribohexopyranosyl) oxy] 6-O-méthyl-3-oxo-12,11-[oxycarbonyl-[[3-(4-quinoléinyl) propyl] hydrazono]] érythromycine.

9. A titre de médicaments, les composés de formule (I) définis à l'une quelconque des revendications 1 à 7.

10. A titre de médicaments, le composé de formule (I) défini à la revendication 8.

11. Les compositions pharmaceutiques renfermant comme principe actif au moins un médicament défini à la revendication 9 ou 10.

12. Procédé de préparation des composés de formule (I) définis à la revendication 1, **caractérisé en ce que** l'on soumet un composé de formule (II) : 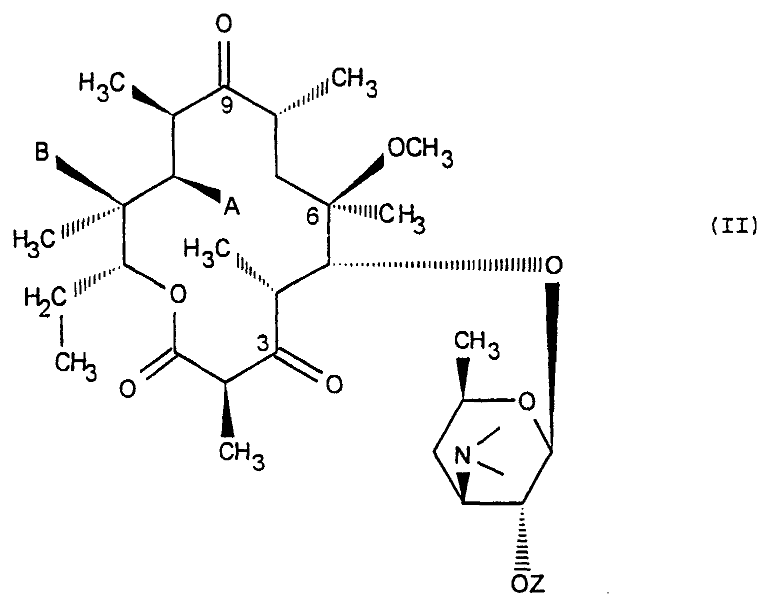 dans lequel A, B et Z conservent leur signification précédente à l'action d'un composé de formule (III) :
HN₂OR
dans lequel R conserve sa signification précédente pour obtenir le composé de formule (I) correspondant, puis si désiré, libère l'hydroxyle en 2' et/ou soumet à l'action d'un acide pour en former le sel, et/ou à l'action d'un agent de modification du radical R.

## Claims

1. The compounds of formula (I): 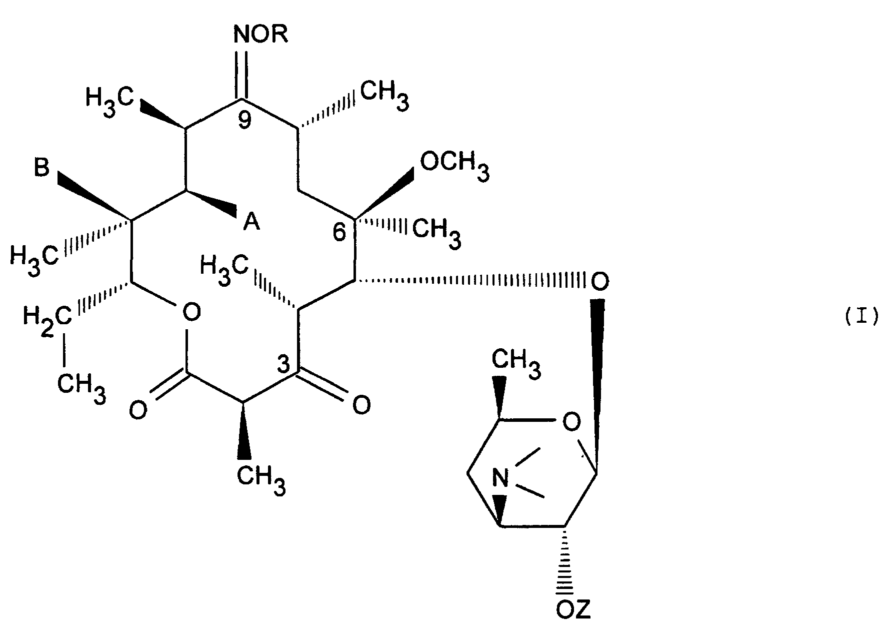 in which A and B form with the atoms to which they are linked a 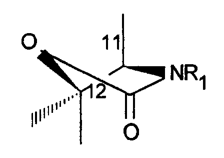 group
- R₁ represents an NH₂ radical or an NH-alkyl, NH-alkenyl or NH-alkynyl radical containing up to 18 carbon atoms or an NH(CH₂)ₙAr or N=CH(CH₂)ₙAr radical in which n represents an integer comprised between 1 and 6, and Ar represents an optionally substituted aryl or heteroaryl radical, Z represents a hydrogen atom or the remainder of a carboxylic acid containing up to 18 carbon atoms,
and R represents:
- a hydrogen atom,
- a heterocyclic radical containing at least one nitrogen atom and optionally another aromatic or non-aromatic, saturated or unsaturated, mono or bicyclic heteroatom comprising up to 12 members, optionally substituted on the nitrogen atom,
- a linear, branched or cyclic, alkyl, alkenyl or alkynyl radical containing up to 18 carbon atoms optionally substituted by one or more groups:
. hydroxyl,
. halogen,
. cyano,
. nitro,
. amidinyl,
. guanidinyl,
. heterocyclic, as defined previously,
. alkyloxy, alkenyloxy or alkynyloxy having at most 6 carbon atoms,
. alkylthio, alkenylthio or alkynylthio having at most 6 carbon atoms, the sulphur atom being optionally oxidized to sulphoxide or to sulphone,
. aryl, aralkyl,
. aryloxy, aralkyloxy,
. arylthio, aralkylthio, the sulphur atom being optionally oxidized to sulphoxide or to sulphone, 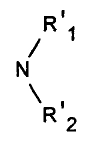 in which:
**either R'**_{**1**} **and R'**_{**2**}**,** identical or different, represent a hydrogen atom, a linear, branched or cyclic alkyl, alkenyl or alkynyl radical containing up to 18 carbon atoms, an aryl or aralkyl radical, each of these R'₁ and R'₂ radicals being optionally substituted by one or more hydroxy, alkyloxy, alkenyloxy, alkynyloxy, alkylthio, alkenylthio or alkynylthio radicals containing up to 8 carbon atoms, amino, monoalkylamino radicals containing up to 4 carbon atoms, dialkylamino radicals containing up to 8 carbon atoms, cyano, free, esterified or salified carboxyl, acyl or carbamoyl radicals, containing up to 8 carbon atoms, by an Si(alk)₃ or Si(Oalk)₃ radical in which alk represents an alkyl radical containing up to 4 carbon atoms, by a heterocyclic radical as defined previously,
**or R'**_{**1**} **and R'**_{**2**} form together with the nitrogen atom to which they are linked a mono or bicyclic heterocycle radical, optionally containing another aromatic or non-aromatic, saturated or unsaturated heteroatom, comprising up to 12 members;
. a quaternary ammonium group,
. 1,2-epoxyethyl or 2,2-dimethyl 1,2-epoxyethyl or a radical resulting from the opening of this group by a nucleophilic reagent, 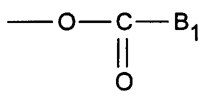 in which B₁ represents either an alkyl or alkyloxy radical having at most 6 carbon atoms, or an aryl, aralkyl, aryloxy or aralkyloxy radical,
. free or protected formyl, free, esterified or salified carboxyl, thiocyanate, acyl or carbamoyl,
. (CH₂)ₙR', R' representing the remainder of an amino acid, and n representing an integer comprised between 0 and 6,
as well as the addition salts with acids of the compounds of formula (I).

2. The compounds of formula (I) as defined in claim 1, in which Z represents a hydrogen atom.

3. The compounds of formula (I) as defined in claim 1 or 2, in which R₁ represents an NH(CH₂)ₙAr radical, n and Ar retaining their meaning indicated in claim 1.

4. The compounds of formula (I) as defined in any one of claims 1 to 4 in which Ar represents an 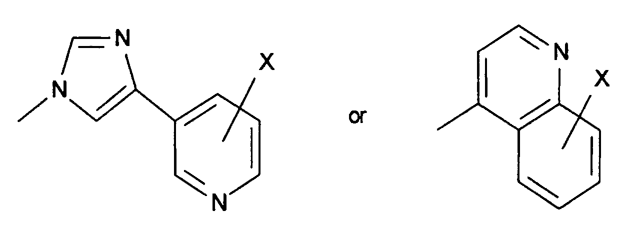 radical and in which X represents a hydrogen atom, a CH₃, OH, OMe radical, or a halogen atom.

5. The compounds of formula (I) defined in claim 4 in which Ar represents an 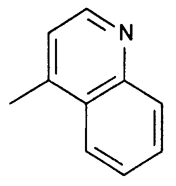 radical.

6. The compounds of formula (I) defined in claim 3, 4 or 5 in which n represents the number 3.

7. The compounds of formula (I) defined in claim 1, in which R represents a hydrogen atom, a 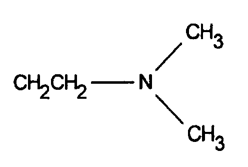 radical
or a 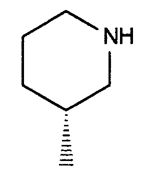 radical optionally substituted on the nitrogen atom by a 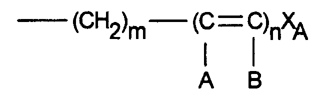 radical in which m represents an integer comprised between 0 and 20, n represents the number 0, 1, 2, or 3,
and either A and B, identical or different, represent a hydrogen atom or a halogen atom or an alkyl or aryl radical, containing up to 8 carbon atoms, the geometry of the double bond being E or Z or an E + Z mixture,
or A and B form a third bond between the carbon atoms to which they are linked,
and X_{A} represents:
. a linear or branched, saturated or unsaturated alkyl radical containing from 6 to 20 carbon atoms, optionally interrupted by one or more heteroatoms and optionally substituted by one or more halogen atoms,
. a cyclic alkyl radical containing from 3 to 8 carbon atoms optionally substituted by a carbocyclic aryl radical,
. a halogen atom,
. a C≡N radical,
. an OR₃, COR₄, CO₂R₅, SR₆, 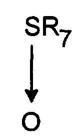 SO₂R₈ or 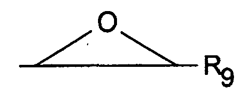 radical in which R₃, R₄, R₅, R₆, R₇, R₈ and R₉ represent a hydrogen atom, an alkyl radical containing up to 8 carbon atoms, optionally interrupted by one or more heteroatoms and optionally substituted by one or more halogen atoms, a carbocyclic or heterocyclic aryl or aralkyl radical containing up to 14 carbon atoms, optionally substituted by one or more radicals chosen from the group constituted by the free, salified, esterified or amidified carboxy radicals, the hydroxy radicals, the halogen atoms, the NO₂ radicals, the C≡N radicals, the alkyl, alkenyl and alkynyl, O-alkyl, O-alkenyl and O-alkynyl, S-alkyl, S-alkenyl and S-alkynyl, SO-alkyl, SO-alkenyl, SO-alkynyl, SO₂-alkyl, SO₂-alkenyl, SO₂-alkynyl radicals, containing up to 12 carbon atoms optionally substituted by one or more halogen atoms, the carbocyclic or heterocyclic aryl, O-aryl, S-aryl radicals containing up to 14 carbon atoms, the 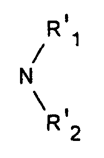 radicals, R'₁ and R'₂, identical or different, representing a hydrogen atom or an alkyl radical containing up to 12 carbon atoms, the aryl radicals themselves optionally substituted by one of the substituents indicated above as substituents of the aryl radicals,
. an NR₁R₂ radical in which either R₁ and R₂, identical or different, represent a hydrogen atom or an alkyl radical containing up to 20 carbon atoms, -CO-alkyl or -CO₂-alkyl radical containing up to 8 carbon atoms, or an aryl, -CO-aryl or -CO₂-aryl aralkyl, -CO-aralkyl or -CO₂-aralkyl radical containing up to 14 carbon atoms, the aryl radicals themselves being optionally substituted by one of the substituents indicated above as substituents of the aryl radicals, or R₁ and R₂ form together with the nitrogen atom to which they are linked a ring with 3 to 8 members optionally comprising another heteroatom and optionally substituted by one of the substituents indicated above as substituents of the aryl radicals,
. a carbocyclic aryl radical optionally substituted by one or more of the radicals stated above as substituents of the aryl radicals,
. a heterocyclic aryl radical comprising one or more heteroatoms, optionally substituted by one or more of the radicals mentioned above as substituents of the aryl radicals,
. an OC(Ar)₃ radical in which Ar represents a carbocyclic aryl radical optionally substituted by one or more of the substituents mentioned above as substituents of the aryl radicals.

8. The compounds of formula (I) defined in claim 1, the name of which follow:
(9E) 11,12-dideoxy-3-de[(2,6-dideoxy-3-C-methyl-3-O-methyl-alpha-L-ribohexopyranosyl) oxy] 6-O-methyl-3-oxo-12,11-[oxycarbonyl-[[3-(4-quinolinyl) propyl] hydrazono]] erythromycin9-[O-(3-piperidinyl) oxime].

9. As medicaments, the compounds of formula (I) defined in any one of claims 1 to 7.

10. As medicaments, the compound of formula (I) defined in claim 8.

11. The pharmaceutical compositions containing at least one medicament defined in claim 9 or 10 as active ingredient.

12. Process for the preparation of the compounds of formula (I) defined in claim 1, **characterized in that** a compound of formula (II): 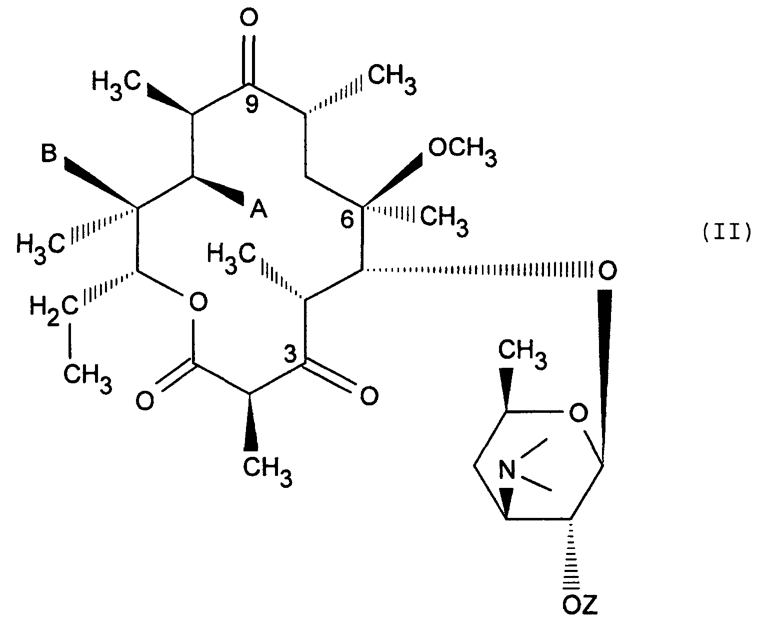 in which A, B and Z retain their previous meaning are subjected to the action of a compound of formula (III):
HN₂OR
in which R retains its previous meaning in order to obtain the corresponding compound of formula (I), then if required, the hydroxyl in position 2' is released and/or subjected to the action of a acid in order to form the salt, and/or to the action of an agent which modifies the R radical.

## Patentansprüche

1. Verbindungen der Formel (I) 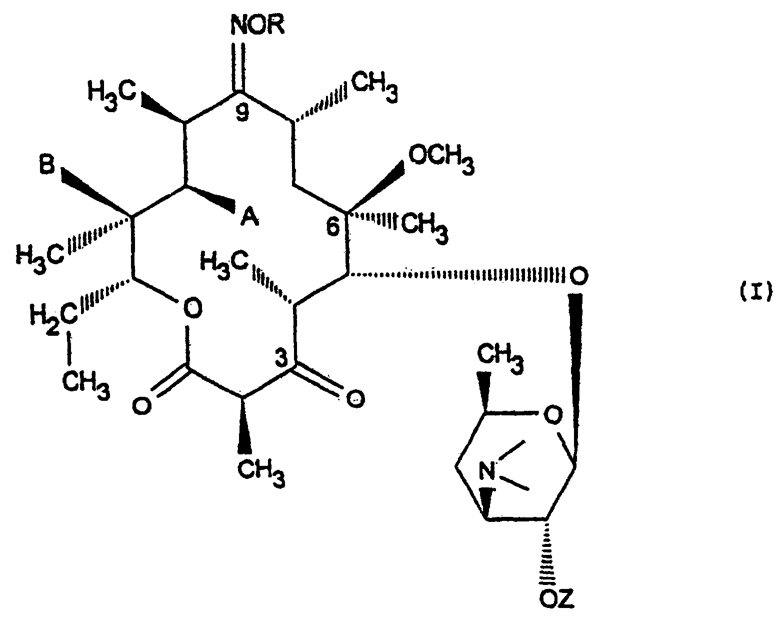 in der A und B zusammen mit den Atomen, an die sie gebunden sind, eine Gruppe 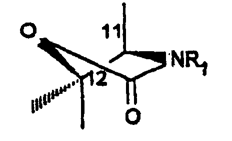 bilden,
- R₁ einen Rest NH₂ oder einen Rest NH-Alkyl, NH-Alkenyl oder NH-Alkinyl mit bis zu 18 Kohlenstoffatomen oder einen Rest NH(CH₂)ₙAr oder N=CH(CH₂)ₙAr darstellt, worin n eine ganze Zahl zwischen 1 und 6 ist und Ar einen Rest Aryl oder Heteroaryl bedeutet, gegebenenfalls substituiert, Z ein Wasserstoffatom oder den Rest einer Carbonsäure mit bis zu 18 Kohlenstoffatomen darstellt und R bedeutet:
- ein Wasserstoffatom,
- einen mono- oder bicyclischen, gesättigten oder ungesättigten, aromatischen oder nicht aromatischen heterocyclischen Rest mit bis zu 12 Ringgliedern und mindestens einem Stickstoffatom und gegebenenfalls einem weiteren Heteroatom, gegebenenfalls substituiert am Stickstoffatom,
- einen linearen, verzweigten oder cyclischen Rest Alkyl, Alkenyl oder Alkinyl mit bis zu 18 Kohlenstoffatomen, gegebenenfalls substituiert durch eine oder mehrere Gruppen:
. Hydroxyl,
. Halogen,
. Cyano,
. Nitro,
. Amidinyl,
. Guanidinyl,
. heterocyclische Gruppen, wie vorstehend definiert,
. Alkyloxy, Alkenyloxy oder Alkinyloxy mit höchstens 6 Kohlenstoffatomen,
. Alkylthio, Alkenylthio oder Alkinylthio mit höchstens 6 Kohlenstoffatomen, wobei das Schwefelatom gegebenenfalls zu Sulfoxid oder zu Sulfon oxidiert ist,
. Aryl, Aralkyl,
. Aryloxy, Aralkyloxy,
. Arylthio, Aralkylthio, wobei das Schwefelatom gegebenenfalls zu Sulfoxid oder zu Sulfon oxidiert ist,
. 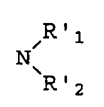 worin: entweder R'₁ und R'₂, gleich oder verschieden, ein Wasserstoffatom, einen linearen, verzweigten oder cyclischen Rest Alkyl, Alkenyl oder Alkinyl mit bis zu 18 Kohlenstoffatomen, einen Rest Aryl oder Aralkyl darstellen, wobei jeder dieser Reste R'₁ und R'₂ gegebenenfalls substituiert ist durch einen oder mehrere Reste Hydroxy, Alkyloxy, Alkenyloxy, Alkinyloxy, Alkylthio, Alkenylthio oder Alkinylthio mit bis zu 8 Kohlenstoffatomen, Amino, Mono- alkylamino mit bis zu 4 Kohlenstoffatomen, Dialkylamino mit bis zu 8 Kohlenstoffatomen, Cyano, Carboxy, frei, verestert oder in Salz überführt, Acyl oder Carbamoyl mit bis zu 8 Kohlenstoffatomen, durch einen Rest Si(alc)₃ oder Si(Oalc)₃, worin alc einen Rest Alkyl mit bis zu 4 Kohlenstoffatomen darstellt, durch einen heterocyclischen Rest wie vorstehend definiert,
oder R'₁ und R'₂ zusammen mit dem Stickstoffatom, an das sie gebunden sind, einen mono- oder bicyclischen, gesättigten oder ungesättigten, aromatischen oder nicht aromatischen heterocyclischen Rest mit bis zu 12 Ringgliedern bilden, der gegebenenfalls ein anderes Heteroatom umfaßt; eine quaternäre Ammoniumgruppe,
1,2-Epoxyethyl oder 2,2-Dimethyl-1,2-epoxyethyl oder einen Rest, der aus der Öffnung dieser Gruppe durch einen nucleophilen Reaktanden resultiert, 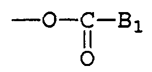 worin B₁ entweder einen Rest Alkyl oder Alkyloxy mit höchstens 6 Kohlenstoffatomen oder einen Rest Aryl, Aralkyl, Aryloxy oder Aralkyloxy darstellt,
. Formyl, frei oder geschützt, Carboxyl, frei, verestert oder in Salz überführt, Thiocyanat, Acyl oder Carbamoyl,
. (CH₂)ₙR', worin R' den Rest einer Aminosäure bedeutet und n eine ganze Zahl zwischen 0 und 6 ist,
sowie die Additionssalze der Verbindungen der Formel (I) mit Säuren.

2. Verbindungen der Formel (I) wie in Anspruch 1 definiert, worin Z ein Wasserstoffatom darstellt.

3. Verbindungen der Formel (I) wie in Anspruch 1 oder 2 definiert, worin R₁ einen Rest NH(CH₂)ₙAr darstellt und n und Ar ihre in Anspruch 1 angegebene Bedeutung beibehalten.

4. Verbindungen der Formel (I) wie in irgendeinem der Ansprüche 1 bis 4 definiert, worin Ar einen Rest 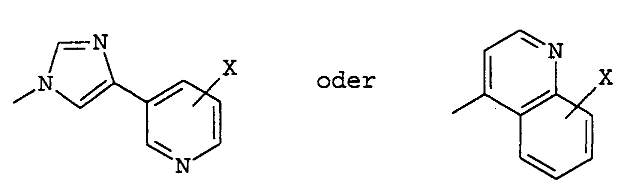 darstellt und worin X ein Wasserstoffatom, einen Rest CH₃, OH, OMe oder ein Halogenatom bedeutet.

5. Verbindungen der Formel (I) wie in Anspruch 4 definiert, worin Ar einen Rest 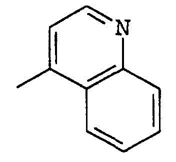 darstellt.

6. Verbindungen der Formel (I) wie in Anspruch 3, 4 oder 5 definiert, worin n die Zahl 3 darstellt.

7. Verbindungen der Formel (I) wie in Anspruch 1 definiert, worin R ein Wasserstoffatom, einen Rest 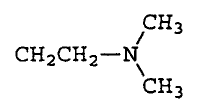 oder einen Rest 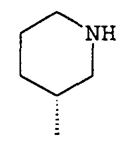 darstellt, gegebenenfalls substituiert am Stickstoffatom durch einen Rest 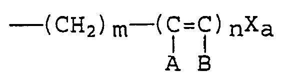 worin m eine ganze Zahl zwischen 0 und 20 darstellt und n die Zahl 0, 1, 2 oder 3 bedeutet,
und entweder A und B, gleich oder verschieden, ein Wasserstoffatom oder ein Halogenatom oder einen Rest Alkyl oder Aryl mit bis zu 8 Kohlenstoffatomen darstellen, wobei die Geometrie der Doppelbindung E oder Z oder eine Mischung E + Z ist,
oder A und B zwischen den Kohlenstoffatomen, an die sie gebunden sind, eine dritte Bindung bilden, und
X_{A} bedeutet:
. einen linearen oder verzweigten, gesättigten oder ungesättigten Rest Alkyl mit 6 bis 20 Kohlenstoffatomen, gegebenenfalls unterbrochen durch ein oder mehrere Heteroatome und gegebenenfalls substituiert durch ein oder mehrere Halogenatome,
. einen cyclischen Rest Alkyl mit 3 bis 8 Kohlenstoffatomen, gegebenenfalls substituiert durch einen carbocyclischen Rest Aryl,
. ein Halogenatom,
. einen Rest C≡N,
. einen Rest OR₃, COR₄, CO₂R₅, SR₆, 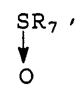 SO₂R₈ oder 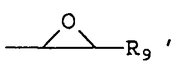 worin R₃, R₄, R₅, R₆, R₇, R₈ und R₉ darstellen: ein Wasserstoffatom, einen Rest Alkyl mit bis zu 8 Kohlenstoffatomen, gegebenenfalls unterbrochen durch ein oder mehrere Heteroatome und gegebenenfalls substituiert durch ein oder mehrere Halogenatome, einen carbocyclischen oder heterocyclischen Rest Aryl oder Aralkyl mit bis zu 14 Kohlenstoffatomen, gegebenenfalls substituiert durch einen oder mehrere Reste, gewählt aus der Gruppe, die gebildet wird durch die Reste Carboxyl, frei, in Salz überführt, verestert oder amidiert, die Reste Hydroxyl, die Halogenatome, die Reste NO₂, die Reste C≡N, die Reste Alkyl, Alkenyl und Alkinyl, O-Alkyl, O-Alkenyl und O-Alkinyl, S-Alkyl, S-Alkenyl und S-Alkinyl, SO-Alkyl, SO-Alkenyl, SO-Alkinyl, SO₂-Alkyl, SO₂-Alkenyl, SO₂-Alkinyl mit bis zu 12 Kohlenstoffatomen, gegebenenfalls substituiert durch ein oder mehrere Halogenatome, die carbocyclischen oder heterocyclischen Reste Aryl, O-Aryl, S-Aryl mit bis zu 14 Kohlenstoffatomen, die Reste 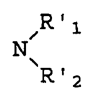 worin R'₁ und R'₂, gleich oder verschieden, ein Wasserstoffatom oder einen Rest Alkyl mit bis zu 12 Kohlenstoffatomen darstellen, wobei die Reste Aryl ihrerseits selbst gegebenenfalls substituiert sind durch einen der oben als Substituenten der Reste Aryl angegebenen Substituenten,
. einen Rest NR₁R₂, worin entweder R₁ und R₂, gleich oder verschieden, ein Wasserstoffatom oder einen Rest Alkyl mit bis zu 20 Kohlenstoffatomen, -CO-Alkyl oder -CO₂-Alkyl mit bis zu 8 Kohlenstoffatomen, oder einen Rest Aryl, -CO-Aryl oder -CO₂-Aryl-aralkyl, -CO-Aralkyl oder -CO₂-Aralkyl mit bis zu 14 Kohlenstoffatomen darstellen, wobei die Reste Aryl ihrerseits selbst gegebenenfalls substituiert sind durch einen der oben als Substituenten der Reste Aryl angegebenen Substituenten,
oder R₁ und R₂ zusammen mit dem Stickstoffatom, an das sie gebunden sind, einen Ring mit 3 bis 8 Ringgliedern bilden, der gegebenenfalls ein anderes Heteroatom umfaßt und gegebenenfalls substituiert ist durch einen der oben als Substituenten der Reste Aryl angegebenen Substituenten,
. einen carbocyclischen Rest Aryl, gegebenenfalls substituiert durch einen oder mehrere der oben als Substituenten der Reste Aryl erwähnten Substituenten,
. einen heterocyclischen Rest Aryl mit einem oder mehreren Heteroatomen, gegebenenfalls substituiert durch einen oder mehrere der oben als Substituenten der Reste Aryl erwähnten Substituenten,
. einen Rest OC(Ar)₃, worin Ar einen carbocyclischen Rest Aryl darstellt, gegebenenfalls substituiert durch einen oder mehrere der oben als Substituenten der Reste Aryl erwähnten Substituenten.

8. Verbindungen der Formel (I) wie in Anspruch 1 definiert, mit den folgenden Namen:
(9E) 9-[O-(3-Piperidinyl)-oxim] von 11,12-Dideoxy-3-de-[(2,6-dideoxy-3-C-methyl-3-O-methyl-alpha-L-ribohexopyranosyl)-oxy]-6-O-methyl-3-oxo-12,11-{oxycarbonyl-[[3-(4-chinolinyl)-propyl]-hydrazono]}-erythromycin.

9. Als Arzneimittel die Verbindungen der Formel (I) wie in irgendeinem der Ansprüche 1 bis 7 definiert.

10. Als Arzneimittel die Verbindung der Formel (I) wie in Anspruch 8 definiert.

11. Pharmazeutische Zusammensetzungen, enthaltend als Wirkstoff mindestens ein Arzneimittel wie in Anspruch 9 oder 10 definiert.

12. Verfahren zur Herstellung der Verbindungen der Formel (I) wie in Anspruch 1 definiert, **dadurch gekennzeichnet, daß** man eine Verbindung der Formel (II) 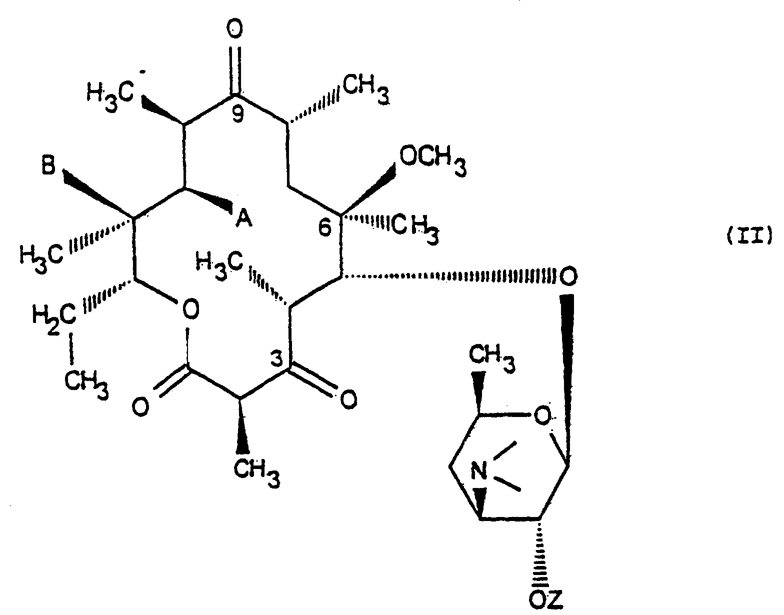 in der A, B und Z ihre vorstehend genannte Bedeutung beibehalten, der Einwirkung einer Verbindung der Formel (III)
HN₂OR (III)
unterzieht, in der R seine vorstehend genannte Bedeutung beibehält, um die entsprechende Verbindung der Formel (I) zu erhalten, bei der man anschließend, wenn erwünscht, das Hydroxyl in 2' freisetzt und/oder die Verbindung der Einwirkung einer Säure unterzieht, um daraus das Salz zu bilden und/oder der Einwirkung eines Mittels zur Modifizierung des Restes R unterwirft.
